# EUROPEAN PATENT APPLICATION

(11) **EP 4 517 767 A1**
(43) Date of publication of application: **05.03.2025**
(21) Application number: 24194375.2
(22) Date of filing: 13.08.2024
(51) Int. Cl.: G16H 20/40, G16H 50/20, A61N 5/10

(54) **EXTERNAL ARTIFICIAL INTELLIGENCE MODEL FOR RADIOTHERAPY PLANNING OPTIMIZATION**

(30) Priority: 28.08.2023 US 202318457237
(71) Applicant: Siemens Healthineers International AG, 6312 Steinhausen (CH)
(72) Inventor: KUUSELA, Esa, 02320 Espoo (FI); BASIRI, Shahab, 02570 Siuntio (FI)
(74) Representative: Mathisen & Macara LLP

(57) **Abstract**

Disclosed herein are methods and systems to evaluate cost values for different radiotherapy treatment plans using external AI models. A method (200) comprises receiving (210) a radiation therapy plan objective for a patient; executing (220) a plan optimizer to generate one or more treatment attributes for a treatment plan complying with the radiation therapy plan objectives, the plan optimizer iteratively calculating the one or more attributes, where with each iteration, the plan optimizer revises the one or more attributes of the treatment plan in accordance with a cost value; executing (230) an AI model to calculate a second cost value for the treatment plan, wherein the AI model is trained to calculate the second cost value in accordance with a likelihood of occurrence of a health-problem for the patient after being treated via the treatment plan having the one or more attributes; and outputting (260) the treatment plan for the patient.

## Description

### TECHNICAL FIELD

This application relates generally to generating, training, and operating artificial intelligence (AI) computer models for loss function predictions for radiation therapy treatment plans.

### BACKGROUND

Radiation therapy (radiation-based therapy or radiotherapy) is a cancer treatment wherein high doses of radiation that can kill cancerous cells or shrink a tumor are applied to the affected area. The target region of a patient's body that is intended to receive radiation (e.g., tumor) is referred to as the planning target volume (PTV). The goal is to deliver enough radiation to the PTV to kill the cancerous cells (also referred to herein as a treatment plan or radiation therapy treatment). However, other organs or anatomical regions that are adjacent to or surround the PTV can be in the path of radiation beams and can receive enough radiation to be damaged or harmed. These organs or anatomical regions are referred to as organs at risk (OARs). Usually, a physician or a radiologist identifies both the PTV and the OARs before radiation therapy by using, for example, computed tomography (CT) images, magnetic resonance imaging (MRI) images, positron emission tomography (PET) images, images obtained via other imaging modalities, or a combination thereof.

Using the medical images of the patient, as well as the identified PTV and the OARs, a team of medical professionals (e.g., physicians, radiologists, oncologists, radiology technicians, other medical personnel, or a combination thereof) determines the radiation parameters to be used during the radiation therapy treatment. These radiation parameters may include, for example, the type, angle, intensity, and/or shape of each radiation beam. In determining these parameters, the team attempts to achieve a radiation dose distribution to the patient that meets predefined criteria (also referred to herein as the plan objectives or clinical objectives).

To optimize the radiation parameters in a manner that meets the predefined criteria, a software solution(s) or a computer model(s) (typically referred to as "plan optimizers") are utilized that can run a plurality of simulations having various radiation parameters and can select, based on the simulation results, a final set of radiation parameters to be used. These radiation parameters are typically referred to as a radiation therapy treatment plan (RTTP).

However, this process can be highly inefficient because plan optimizers typically use an iterative, trial-and-error process to determine various attributes of the RTTP. For instance, after the treating physician inputs the plan objectives, the software solution iteratively analyzes different possibilities (e.g., different iterations of varying attributes within a large search space) to identify one or more attributes of the RTTP. As a result, the software solutions may require substantial computing resources and may not produce timely results. Also, various long-term effects may not be readily apparent to the plan optimizer, and/or the plan optimizer may not have the capacity to account for long-term effects when generating the RTTP.

### SUMMARY

In accordance with a first aspect of the invention, there is provided a method as defined by claim 1.

In accordance with a second aspect of the invention, there is provided a non-transitory machine-readable storage medium as defined by claim 6.

In accordance with a third aspect of the invention, there is provided a system as defined by claim 11.

Optional features are defined by the dependent claims.

For the aforementioned reasons, there is a desire for an external computer model to analyze the long-term effects of an RTTP using methods and systems that are not dependent on static/internal cost functions of a plan optimizer computer model. There is a desire for an improved AI modeling/training technique that does not require extensive reconfiguration of the plan optimizer computer model and is computationally efficient, cost-effective, and produces timely results.

RTTP creation is typically based on the optimization of certain dosimetrical parameters, such as "target coverage," "max dose in spinal cord," and/or "mean dose in parotid gland." The used dosimetrical parameters and the desired goal values are often based on previously performed outcome studies where there is a correlation between these dosimetrical parameters and certain clinical endpoints. The observed correlation between dose distribution and clinical outcomes is sometimes referred to as the "clinical models." Conventional plan optimizers may optimize the RTTP against a given clinical model by defining the desired objectives, but they are bound to use certain generic dosimetrical parameters, which were implemented and sometimes hardcoded within the software algorithm during the product development (such as "Dose-to-Volume," "Volume-to-Dose," and/or "Generalized Equivalent Uniform Dose." This static approach is undesirable because it is time-consuming and computationally intense to revise the software code.

With the rapid pace of innovation in the field of radiotherapy planning, it is expected that more complicated spatial prediction models are going to be created in the near future. For instance, imaging technology is improving to the point that creating a new type of spatial functional images might be possible. Moreover, the current rapid development of machine learning techniques, such as neural networks may allow the creation of more accurate spatial prediction models. As a result, implementing the conventional static approach (e.g., where new 'dosimetrical' parameters need to be implemented as part of the software code to support new prediction models) will become increasingly computationally expensive.

In an embodiment, a method may comprise receiving, by a processor, a radiation therapy plan objective for a patient; executing, by the processor, a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value; executing, by the processor, an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and outputting, by the processor, the treatment plan for the patient. For example, the health-problem may be a side effect or a consequence of the treatment plan.

The method may further comprise transmitting, by the processor, the second cost value to the plan optimizer computer model, wherein the plan optimizer computer model revised, or revises, the one or more attributes of the treatment plan in accordance with the second cost value.

The health-problem may correspond to at least one of xerostomia, reduction of saliva production, headache, hair loss, nausea, vomiting, fatigue, hair loss, skin irritation, memory loss, or speech loss.

The radiation therapy plan objective may correspond to a dose-volume objective.

The health-problem may correspond to developing a secondary cancer.

The plan optimizer computer model may aggregate the second cost value generated by the artificial intelligence model with the cost value generated by the plan optimizer computer model.

The artificial intelligence model may be customized for a clinic implementing the treatment plan for the patient.

In another embodiment, a non-transitory machine-readable storage medium has computer-executable instructions stored thereon that, when executed by one or more processors, may cause the one or more processors to perform operations comprising: receive a radiation therapy plan objective for a patient; execute a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value; execute an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and output the treatment plan for the patient.

The instructions may further cause the one or more processors to transmit the second cost value to the plan optimizer computer model, wherein the plan optimizer computer model revised, or revises, the one or more attributes of the treatment plan in accordance with the second cost value.

The health-problem may correspond to at least one of xerostomia, reduction of saliva production, headache, hair loss, nausea, vomiting, fatigue, hair loss, skin irritation, memory loss, or speech loss.

The radiation therapy plan objective may correspond to a dose-volume objective.

The health-problem may correspond to developing a secondary cancer.

The plan optimizer computer model may aggregate the second cost value generated by the artificial intelligence model with the cost value generated by the plan optimizer computer model.

The artificial intelligence model may be customized for a clinic implementing the treatment plan for the patient.

In another embodiment, a system may comprise at least one processor configured to: receive a radiation therapy plan objective for a patient; execute a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value; execute an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and output the treatment plan for the patient.

The at least one processor may be further configured to transmit the second cost value to the plan optimizer computer model, wherein the plan optimizer computer model revised, or revises, the one or more attributes of the treatment plan in accordance with the second cost value.

The health-problem may correspond to at least one of xerostomia, reduction of saliva production, headache, hair loss, nausea, vomiting, fatigue, hair loss, skin irritation, memory loss, or speech loss.

The radiation therapy plan objective may correspond to a dose-volume objective.

The health-problem may correspond to developing a secondary cancer.

The plan optimizer computer model may aggregate the second cost value generated by the artificial intelligence model with the cost value generated by the plan optimizer computer model.

The artificial intelligence model may be customized for a clinic implementing the treatment plan for the patient.

In one example, the treatment plan generated by the plan optimizer computer model may be inputted to the artificial intelligence model which may then calculate a cost value relating to a side effect caused by carrying out the treatment plan on the patient.

The cost value relating to the side effect may then be inputted to the plan optimizer computer model which may then generate a revised treatment plan.

The revised treatment plan may then be inputted to the artificial intelligence model which may then calculate a revised cost relating to the side effect caused by carrying out the revised treatment plan on the patient.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of an AI-enabled RTTP generation system, according to an embodiment.
**FIG. 2** illustrates a flow diagram of a process executed in an AI-enabled RTTP generation system, according to an embodiment.
**FIG. 3** illustrates embodiments of calculating a cost value, according to different embodiments.
**FIG. 4** illustrates different flow diagrams of different processes executed in an AI-enabled RTTP generation system, according to an embodiment.

### DETAILED DESCRIPTION

Reference will now be made to the illustrative embodiments depicted in the drawings, and specific language will be used herein to describe the same. It will nevertheless be understood that no limitation of the scope of the claims or this disclosure is thereby intended. Alterations and further modifications of the inventive features illustrated herein, and additional applications of the principles of the subject matter illustrated herein that would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting to the subject matter presented.

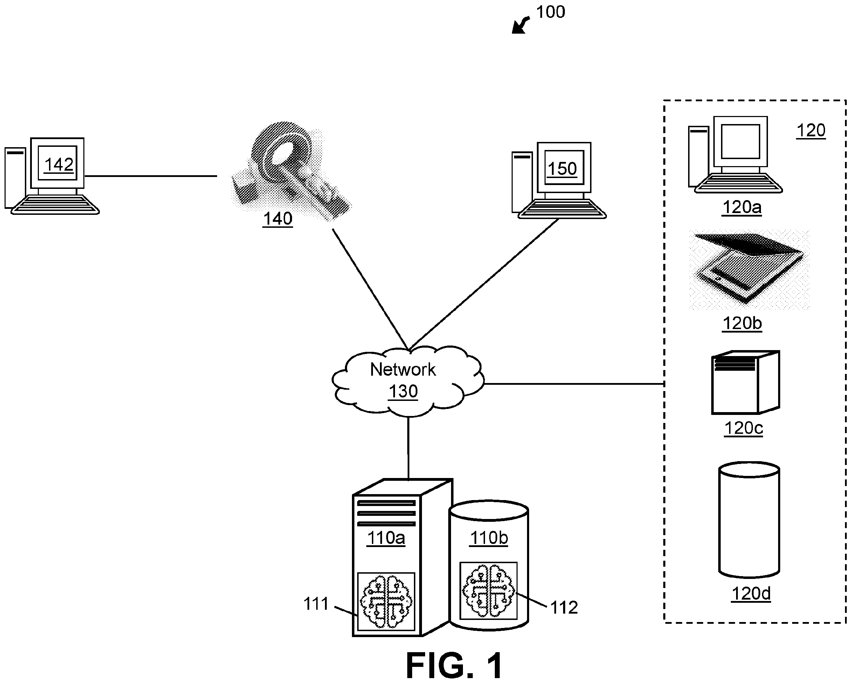

**FIG. 1** illustrates components of an AI-enabled RTTP generation system (system **100**), according to an embodiment. The system **100** may include an analytics server **110a,** system database **110b**, clinical model analysis computer model **111,** end-user devices **120a-d** (collectively end-user devices **120**), and an administrator computing device **150.** Various components depicted in **FIG. 1** may belong to a radiation therapy clinic at which patients may receive radiation therapy treatment via one or more radiation therapy machines (e.g., medical device **140**) in the clinic. The above-mentioned components may be connected through a network **130.** Examples of the network **130** may include, but are not limited to, private or public LAN, WLAN, MAN, WAN, and the Internet. The network **130** may employ wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, such as a GSM (Global System for Mobile Communications), CDMA (Code Division Multiple Access), or EDGE (Enhanced Data for Global Evolution) network.

The system **100** is not confined to the components described herein and may include additional or other components, not shown for brevity, which is to be considered within the scope of the embodiments described herein.

The analytics server **110a** may generate and display an electronic platform configured to use various computer models **111-112** to identify attributes of an RTTP for a patient's treatment. The electronic platform may include a graphical user interface (GUI) displayed on the end-user devices **120** and/or the administrator computing device **150.** An example of the electronic platform generated and hosted by the analytics server **110a** may be a web-based application or a website configured to be displayed on different types of electronic devices, such as mobile devices, tablets, personal computers, and the like.

In a non-limiting example, a physician operating the physician device **120b** may access the platform, input patient attributes, characteristics, and other data, and further instruct the analytics server **110a** to generate an RTTP (including the beam angle). Additionally or alternatively, the analytics server **110a** may optimize only one particular attribute of the RTTP (e.g., optimize beam angle only).

The analytics server **110a** may recommend the RTTP (e.g., beam angles and other radiation parameters and/or treatment plan attributes) used for proton radiation, photon radiation, and electron radiation. In particular, analytics server **110a** may utilize the methods and systems described herein to execute the clinical model analysis computer **111** for identifying cost values that can ultimately be used (e.g., by the plan optimizer model **112**) to evaluate and re-evaluate the RTTP. Further, the analytics server **110a** may transmit the beam angles and other radiation parameters and/or treatment plan attributes to one or more other servers. Additionally or alternatively, the analytics server **110a** (or another server) may adjust the configuration of one or more devices (e.g., the medical device **140**) based on the optimized beam angle. For instance, the RTTP may be directly sent to the medical device **140** and/or the computer **142** that functionally controls the medical device **140.**

The medical device **140** may be any medical device used in the radiation therapy treatment of a patient (such as a CT scan machine, radiation therapy machine (e.g., a linear accelerator, particle accelerator (including circular accelerators), or cobalt machine)). The medical device **140** may also include an imaging device capable of emitting radiation such that the medical device **140** may obtain images according to various methods to accurately image the internal anatomical structure of a patient. For instance, the medical device **140** may include a rotating system (e.g., a static or rotating multi-view system). A non-limiting example of a multi-view system may include stereo systems (e.g., two systems arranged orthogonally).

The analytics server **110a** may host a website accessible to users operating any of the electronic devices described herein (e.g., end-users or medical professionals), where the content presented via the various webpages may be controlled based upon each particular user's role or viewing permissions. The analytics server **110a** may be any computing device comprising a processor and non-transitory, machine-readable storage capable of executing the various tasks and processes described herein. The analytics server **110a** may employ various processors such as central processing units (CPU) and graphics processing units (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various webpages to each end-user devices **120.** Different users may use the website to view and/or interact with the recommended (optimized) results. Different servers, such as a clinic server **120c** may also use the recommended results in downstream processing.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly identifying the user) match an appropriate set of credentials that authenticate the user.

The analytics server **110a** may generate and host webpages based upon a particular user's role within the system **100.** In such implementations, the user's role may be defined by data fields and input fields in user records stored in the system database **110b.** The analytics server **110a** may authenticate the user and may identify the user's role by executing an access directory protocol (e.g., LDAP). The analytics server **110a** may generate webpage content that is customized according to the user's role as defined by the user record in the system database **110b.**

The analytics server **110a** may receive various clinical objectives, patient data, and treatment data from the end-user devices **120.** For instance, a physician may access the platform provided by the analytics server **110a** using a physician device **120b.** The physician may input various patient attributes and/or clinical objectives using one or more input elements of the platform. The analytics server **110a** may then execute various methods discussed herein and display an RTTP on the platform.

The end-user devices **120** may be any computing device comprising a processor and a non-transitory machine-readable storage medium capable of performing the various tasks and processes described herein. Non-limiting examples of end-user devices **120** may be a workstation computer, laptop computer, tablet computer, and server computer. During operation, various users may use end-user devices **120** to access the GUI operated by the analytics server **110a.** Specifically, the end-user devices **120** may include a clinic computer **120a,** a physician device **120b,** a clinic server **120c,** or a clinic database **120d.**

In order to generate the RTTP, the analytics server **110a** may then execute various models (e.g., clinical model analysis computer model **111** and/or a plan optimizer model **112**) to analyze the retrieved/received data.

The administrator computing device **150** may represent a computing device operated by a system administrator. The administrator computing device **150** may be configured to display beam angles, radiation parameters, and/or other radiation therapy treatment attributes generated by the analytics server **110a;** monitor the clinical model analysis computer model **111** utilized by the analytics server **110a** and/or end-user devices **120;** review feedback; and/or facilitate training or retraining (calibration) of the models **111-112** that are maintained by the analytics server **110a.**

The analytics server **110a** may be in communication (real-time or near real-time) with the medical device **140** (or its computer **142**), such that a server/computer hosting the medical device **140** can adjust the medical device **140** based on the RTTP (e.g., beam angles, treatment attributes and/or radiation parameters determined by the analytics server **110a**). For instance, the radiation therapy machine may adjust the gantry, beam blocking device (e.g., multi-leaf collimator MLC), and couch based on optimized beam angles, where the optimized beam angle is an angle of the medical device **140** that emits radiation in a direction of the PTVs. The analytics server **110a** may transmit instructions to the radiation therapy machines indicating any number or type of radiation parameters and/or treatment attributes to facilitate such adjustments.

The clinical model analysis computer model **111** and/or **112** may represent any collection of algorithmic logic and/or artificial intelligence models (e.g., using various machine learning techniques). For instance, the clinical model analysis computer model **111** may include various algorithms to identify a cost value for an RTTP based on patient data and/or treatment attributes and how they would likely cause a secondary and/or long term effect (e.g., health problem). Using the clinical model analysis computer model **111,** the analytics server **110a** may execute the plan optimizer model **112.** For instance, the analytics server **110a** may execute the models in conjunction with each other and then revise one or more configuration parameters of the plan optimizer **112.** As a result, the efficiencies discussed herein can be achieved without revising the plan optimizer **112** itself. For instance, the clinical model analysis computer model **111** can be executed independently and used in conjunction with an existing plan optimizer. Therefore, the plan optimizer (or the system infrastructure) can be retrofitted using the methods and systems discussed herein. This minimal interference with existing infrastructure allows for the improvement of a plan optimizer without the need to revise its source code.

In some embodiments, the analytics server **110a** may collect patient data from various sources not shown in **FIG. 1****.** For instance, the analytics server **110a** may monitor and collect patient and treatment attributes associated with previously-treated radiotherapy patients to include in a training dataset. Additionally or alternatively, the analytics server **110a** may collect/aggregate other patient data (e.g., data associated with how a set of patients or clinical participants received their respective radiotherapy treatment). Using the collected data, the analytics server **110a** may generate a training dataset with which the analytics server **110a** can train the clinical model analysis computer model **111.** As a result, the clinical model analysis computer model **111** may be used to limit the search space used by the plan optimizer **112.**

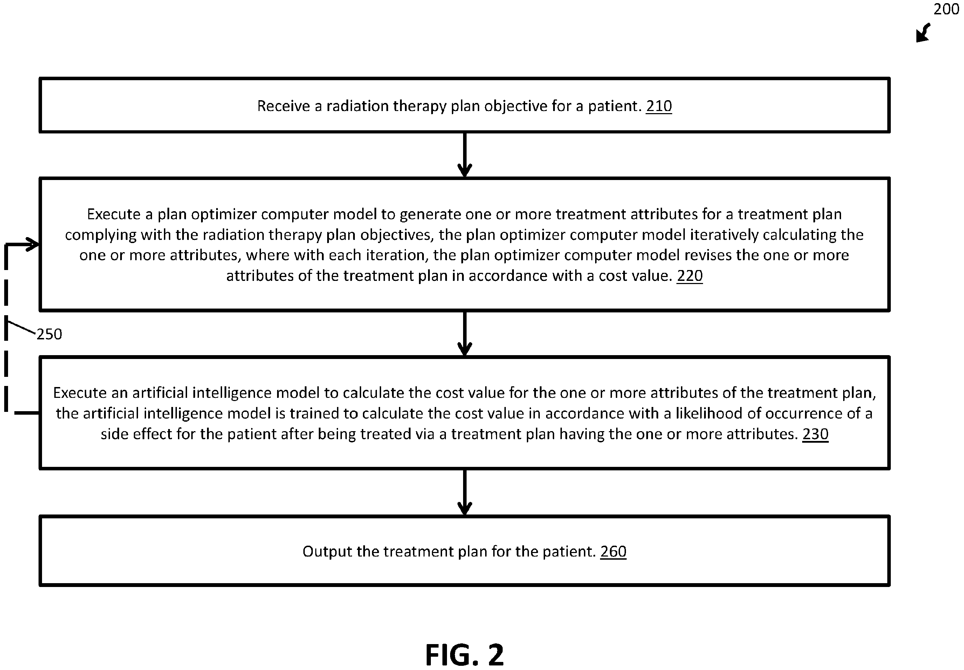

**FIG. 2** illustrates a flow diagram of a process executed in an AI-enabled RTTP generation system, according to an embodiment. The method **200** includes steps **210-260.** However, other embodiments may include additional or alternative execution steps or may omit one or more steps altogether. The method **200** describes how an Al model may be trained and then executed, such that it can predict a cost value for an RTTP.

One or more steps of method **200** may be executed by any number of computing devices operating in the distributed computing system described in **FIG. 1****.** For instance, a different processor (or even a third-party processor) may train the artificial intelligence model than the processor executing the same artificial intelligence model. Moreover, the artificial intelligence model may be executed in conjunction with a plan optimizer that is being executed for and/or by a third party. Therefore, even though the method **200** is described as being executed/implemented by the analytics server, it is possible that each step of the method **200** is executed for a different entity/processor.

At step **210,** the analytics server may receive a radiation therapy plan objective for a patient. The analytics server may receive various attributes determined by a treating physician or a clinician for a patient's radiotherapy treatment. These attributes may be determined for the patient based on the patient's treatment and are generally referred to as plan objectives. A non-limiting example of a plan objective may include dose limits where a treating physician identifies the dosage that needs to be applied to the tumor.

The analytics server may receive and/or retrieve (via a platform and/or via querying a database) patient data needed to calculate a treatment plan for the patient. Therefore, as used herein, patient data may refer to any data that is associated with the patient (e.g., the patient's physical data, diagnosis data, and any data inputted by a medical professional). In some embodiments, the analytics server may use different patient identifiers to retrieve the patient data. For instance, the analytics server may query one or more databases to identify medical data associated with the patient. The analytics server may query data associated with the patient's anatomy, such as physical data (e.g., height, weight, and/or body mass index) and/or other health-related data (e.g., blood pressure or other data relevant to receiving radiation therapy treatment). The analytics server may also retrieve data associated with current and/or previous medical treatments received by the patient (e.g., data associated with the patient's previous surgeries).

In some embodiments, the analytics server may also analyze the patient's medical data records to identify the patient attributes needed. For instance, the analytics server may query a database to identify the patient's BMI. However, because many medical records are not digitized, the analytics server may not receive the patient's BMI value using simple query techniques. As a result, the analytics server may retrieve the patient's electronic health data and may execute one or more analytical protocols (e.g., natural language processing) to identify the patient's body mass index. In another example, if the analytics server does not receive tumor data (e.g., end-points) the analytics server may execute various image recognition protocols and identify the tumor data.

The analytics server may also receive additional data from one or more medical professionals. For instance, a treating oncologist may access a platform generated/hosted by the analytics server and may add, remove, or revise data associated with a particular patient, such as patient attributes, treatment attributes, tumor attributes, the primary site of treatment, tumor stage, endpoints, whether the primary tumor has been extended, and the like. Because tumor staging and the end-level attributes are critical pieces of information that affect patient treatment, this information is typically inputted by the treating oncologist. In some embodiments, an AI model (e.g., a separate Al model that is trained to identify tumor information) may identify this information and the treating oncologist may deny, approve, or revise the predicted results. In another example, the treating oncologist may specifically indicate whether the treatment should be unilateral or bilateral.

Another example of plan objective and/or patient data/attributes may include specific tumor locations. More specifically, this data may indicate the primary tumor location with respect to the patient's centerline. This data may be inputted by the treating oncologist or may be analyzed using various image recognition or segmentation methods executed on the patient's medical images. In some embodiments, this information can also be predicted using the AI model if it is not inputted by the treating oncologist (or otherwise received by the analytics server). Another patient attribute may indicate whether and how close the tumor is to other non-diseased organs. For instance, a tumor to be eradicated may be millimeters away from another organ. This information may change field geometry (generated by the optimizer), as other organs must be avoided.

At step **220,** the analytics server may execute a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value.

The analytics server may deploy a plan optimizer computer model to generate an RTTP for the patient based on the patient data. The plan optimizer may iteratively calculate one or more attributes of the RTTP. For instance, with each iteration, the plan optimizer computer model may generate a candidate RTTP having various attributes. The plan optimizer computer model may then use one or more loss functions to calculate a cost value for the generated candidate RTTP. The cost value may indicate a likelihood of the candidate RTTP violating a set of rules, whether internal and/or external rules. For instance, the cost value may indicate whether the candidate RTTP violates any of the plan objectives (e.g., received in the step 210). The plan optimizer computer model may then analyze the cost value. If needed, e.g., when the cost value satisfies a threshold, the plan optimizer computer model may revise the candidate RTTP and re-execute its loss function to generate a new cost value. Depending on whether the new cost function is increasing or decreasing, the plan optimizer computer model may revise the candidate RTTP again and recalculate the cost value. The plan optimizer computer model may continue this iterative approach until it converges upon an RTTP (or the final RTTP) that has a cost value that satisfies a threshold.

At step **230,** the analytics server may execute an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes. For example, the artificial intelligence model may calculate the second cost value in accordance with a likelihood of occurrence of a health-problem for the patient, wherein the health-problem would arise as a consequence of being treated via the treatment plan.

The artificial intelligence model may comprise neural networks (NN) trained to predict certain clinical outcomes. Specifically, the artificial intelligence model may determine a cost value (other than the one generated by the plan optimizer computer model) for the RTTP that indicates a likelihood of the patient developing a health problem (e.g., side effect) from being potentially treated using the attributes within the RTTP generated by the plan optimizer computer model. In some embodiments, the neural network architecture and weights can be loaded by the plan optimizer computer model as a file, so it may not need to be a part of the software release or be a part of the plan optimizer computer model itself. Accordingly, the methods and systems discussed herein allow for an independent artificial intelligence model to be implemented in conjunction with a plan optimizer computer model without requiring the two separate models to be reconfigured. For example, the artificial intelligence model determines a cost associated with a side effect of a treatment plan and this cost may then be used by a conventional plan optimizer computer model in revising the treatment plan.

This method allows the artificial intelligence model to improve the results as a retroactive solution requiring minimal technical knowledge. Moreover, the methods and systems discussed herein allow for the artificial intelligence model to be plan-optimizer-agnostic where the artificial intelligence model is compatible with any plan optimizer computer model as long as it can ingest the results outputted/predicted by the plan optimizer computer model. For instance, the artificial intelligence model may generate a file or other data format type, such that the evaluation of the plan optimizer computer model can be done in a generic software component.

The artificial intelligence model may predict a cost value that corresponds to the high-level utility of certain predicted probabilities, such as a probability of one or more side effects of other medical issues occurring. The cost value may correspond to a likelihood of the RTTP causing a health-related issue, such as long term side effects or other health issues discussed herein. As used herein the second cost value or the cost value calculated by the artificial intelligence model may refer to any data associated with the cost value used by the plan optimizer. For instance, in some embodiments, the artificial intelligence model may calculate the actual cost value that can be directly used by the plan optimizer computer model. Additionally or alternatively, the artificial intelligence model may generate one or more scores that can then be used to calculate the cost value. For instance, the values predicted by the artificial intelligence model may be transformed (e.g., using other algorithms) to the cost value. Accordingly, the artificial intelligence model can be trained to predict one or more attributes which can then be converted into a cost value that is used by the plan optimizer. The value predicted by the artificial intelligence model can be converted/transformed into a cost value using a conventional function. In a non-limiting example, the cost value (second cost) predicted by the artificial intelligence model may be a likelihood of headache, which can then be used to calculate a cost function used by the plan optimizer.

The input to the artificial intelligence model may consist of a set of channels describing different (independent) features of the finalized and/or candidate RTTPs generated by the plan optimizer computer model. Non-limiting examples of the input to the artificial intelligence model may include dose distribution (of the current candidate RTTP, some other similar information such as "2Gy equivalent dose" or "proton-LET," and/or some dose-rate corrected dose distribution trying to capture Flash effect), target or Organ-at-Risk masks, and/or CT or other imaging modalities.

In some embodiments, when the artificial intelligence model is used as a part of the cost function for the plan optimizer computer model, the user may be required to describe what channels are/should be used as input. In some embodiments, the channels can be deduced automatically by the plan optimizer computer model and/or the artificial intelligence model based on the type of optimizer data objects.

In some embodiments, the plan optimizer may also transmit functional images to the artificial intelligence model (as an input channel). In some embodiments, the particular imaging modality may not be known to the optimizer computer model and its interpretation may be dependent only on the specified artificial intelligence model. In addition, the input channels may need to be in a certain spatial resolution and size. Accordingly, the models discussed herein can contain a component that checks that the dimensionality and resolution of the input data (such as dosage) are set correctly, such that the image can be ingested by the artificial intelligence model.

Using the methods and systems discussed herein allows user-specified artificial intelligence models to be a part of the cost function and further allows the support of new kinds of spatial prediction models without the need to release new optimizer algorithms. It also allows users to use any other spatial information to support the spatial clinical model evaluation.

Before the artificial intelligence model is executed to calculate a cost value, the analytics server may train the artificial intelligence using a training dataset that includes training data comprising previously treated patients and/or patients who participated in one or more clinical trials. The analytics server may monitor and record health-related problems (after their treatment, the health problems for example arising as a consequence of their treatment) for the patients who have been treated. For instance, patients may be evaluated against set schemas regarding side effects and/or known health-related issues. Non-limiting examples of these health-related issues may include xerostomia; headaches; hair loss; nausea; vomiting; extreme tiredness (fatigue); hearing loss or impairment; skin and/or scalp issues (changes), trouble with memory and/or speech, sore skin, diarrhea, fertility issues, and the like. When a health-related problem is identified, human reviewers (or sometimes using a processor that uses pre-set rules), can quantify the health-related problem, such that the quantified health-related problem and its corresponding category can be included within the training dataset. Moreover, the health-related problem may include the development of secondary cancer. The training dataset may also include a timeline associated with each health-related problem.

The training dataset may also include data associated with the patient and their specific RTTP. For instance, for each patient, the training dataset may include patient data (e.g., all available data associated with the patient's anatomy, disease, diagnoses, and any other data ingested by the plan optimizer at the time the patient was treated), any data available that was inputted by the patient's treating physician, and/or the patient's RTTP and its attributes. That is, the training dataset may include each patient's information regarding their anatomy and disease; how the patient was diagnosed; how the patient was treated; and how the patient experienced (e.g. consequential) health-related issues and a corresponding timeline.

Using this training dataset, the analytics server may train the artificial intelligence model, such that the artificial intelligence model can ingest data associated with a new patient and the new patient's RTTP to predict a likelihood of a health-related issue occurring (e.g. as a consequence of performing the RTTP on the new patient). The training may be conducted using a supervised, unsupervised, or semi-supervised manner.

When the training is completed, the artificial intelligence model can be used to generate a new cost value for an RTTP. Specifically, when the plan optimizer generates an RTTP or a candidate RTTP, the artificial intelligence model may ingest the RTTP, patient information, and other pertinent data to predict the likelihood that the patient would (e.g. consequentially) develop one or more health-related issues if the patient is treated using that RTTP or the candidate RTTP.

In some embodiments, the training of the artificial intelligence model may be specific to a clinic or a particular region. For instance, certain clinics may have specific rules and tolerances that are unique to them. Therefore, the analytics server may customize the artificial intelligence model to predict a cost value that complies with the clinic's particular requirements, tolerances, and rules. Moreover, certain artificial intelligence models or sub-models may be customized for a specific health-related problem. For instance, a sub-model may be trained to predict the likelihood of secondary cancer and another sub-model may be trained to predict the likelihood of xerostomia occurring.

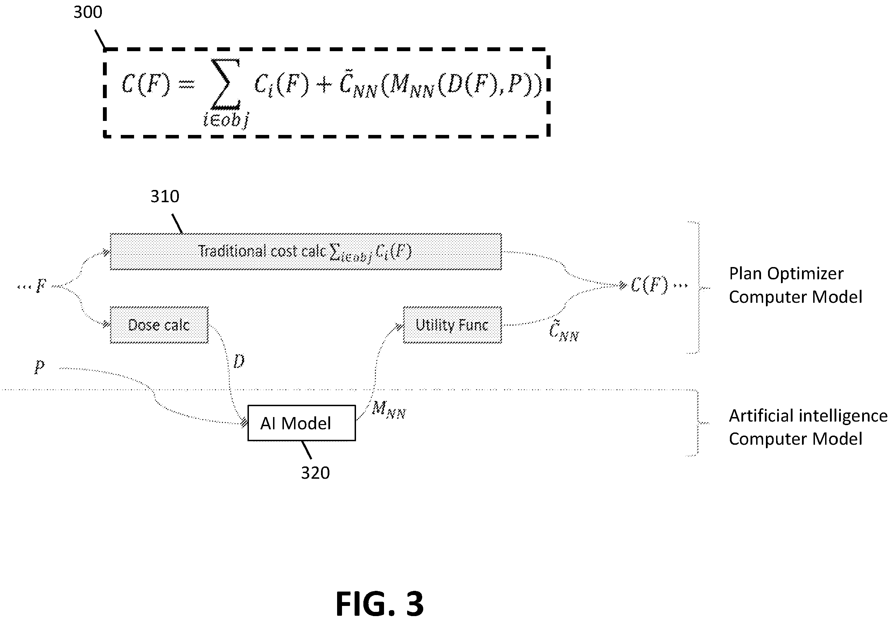

In some embodiments, the prediction of a cost value may be bifurcated among the conventional/traditional calculations and the use of the artificial intelligence computer model discussed herein. The analytics server may then aggregate the cost value generated using the two different methods. For instance, to calculate the cost function, the artificial intelligence may use paradigm **300** illustrated in **FIG. 3****.** As illustrated, the cost function can be bifurcated into the traditional cost calculation **310** that is evaluated entirely as part of the optimizer algorithm used by the plan optimizer computer model; and the artificial intelligence model-dependent **320** (e.g., *C̃_{NN}*(*M_{NN}*(*D*(*F*), *P*))).

When calculating the cost value, the artificial intelligence model is assumed to be depending on the degrees of freedom (*F*). This value may be optimized to improve the RTTP in light of the dose matrix (*D*). Therefore, as illustrated, the traditional cost calculation may emphasize how the RTTP performs in terms of optimizing and not violating dose-volume objectives. Non-limiting examples may include fluence pixel values and/or machine control points.

In the above-described formula, *P* may represent organ masks or functional images. The metric provided by the artificial intelligence model may then be sent back to the high-level utility function (*C̃_{NN}*) containing, for example, a goal level for the metric or parameters describing its importance relative to other plan quality metrics. In generating an RTTP, not all variables may be treated equally. Therefore, certain attributes or objectives may be weighted higher than other objectives. For instance, dose-objectives (and their corresponding negative impact of possible violations) may be treated as more important (having a higher weight) than hair loss of a patient, as a side effect.

Referring back to **FIG. 2****,** at step **250,** the analytics server may optionally transmit the cost value predicted by the artificial intelligence model back to the plan optimizer computer model, such that the plan optimizer computer model can revise the RTTP or candidate RTTP accordingly.

At step **260,** the analytics server may output the treatment plan for the patient. When the RTTP is finalized, the analytics server may output (e.g., display on a platform) the RTTP and its various attributes for the user (e.g., clinicians or treating physicians). In some embodiments, the analytics server may directly instruct a radiotherapy machine to change one or more configurations by the finalized RTTP.

In a non-limiting example, a treating physician inputs a patient's diagnosis and plan objective using one or more input elements received via an electronic platform. The analytics server may then receive a request to generate an RTTP for the patient using a plan optimizer computer model. The analytics server may further retrieve patient information and any other data needed to execute the plan optimizer computer model to generate the RTTP. As a result, the plan optimizer computer model ingests the aggregated data and generates a candidate RTTP that satisfies various cost functions internal to the plan optimizer. The RTTP and the patient information are then ingested by the artificial intelligence model where the artificial intelligence model predicts that the patient may develop a side effect (e.g. the patient will likely (60% chance) develop secondary cancer) as a result of being treated by this RTTP, e.g. within 6-8 months after treatment. The predicted cost value/likelihood may then be ingested back by the plan optimizer computer model where the RTTP is further revised accordingly and a second RTTP is generated. The second RTTP may then be ingested by the artificial intelligence model and the artificial intelligence model may determine a revised side effect (e.g. that the chance of secondary cancer is reduced to 5%), which may then be identified to be within tolerable thresholds. As a result of the side effect being within tolerable thresholds, the analytics server may transmit the second RTTP to the platform and may present the second RTTP for the clinicians/treating physician.

It should be understood that when the AI-predicted attribute (cost) is ingested back to the plan optimizer computer model, the cost value may be one or many factors considered by the plan optimizer. Therefore, the cost value may be among many other optimization attributes in generating the second RTTP. Accordingly, the second RTTP depends on how what solution (trade-off) the plan optimizer implements. Development of the second RTTP may depend on the importance of the cost value predicted by the artificial intelligence model. For example, in determining the second RTTP, the cost value predicted by the artificial intelligence model may be ignored when the cost value relates to relatively unimportant side effects. In determining the second RTTP, the cost value predicted by the artificial intelligence model may be used when the cost value relates to relatively important side effects. For instance, in some embodiments, the cost value predicted by the artificial intelligence model may be ignored (e.g., when the cost value of the artificial intelligence model indicates a high likelihood of headache). In some embodiments, the cost value (e.g., when it indicates a high likelihood of secondary cancer) may be highly impactful to the second RTTP.

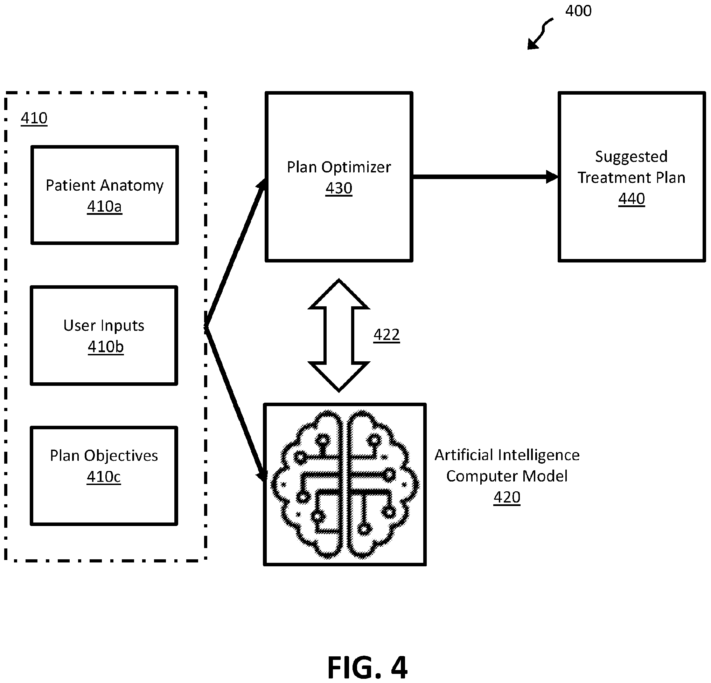

Referring now to **FIG. 4****,** a non-limiting visual example of a workflow utilizing the methods and systems described herein is illustrated. In this non-limiting example **400,** the analytics server provides plan objectives **410c,** patient anatomy **410a,** user inputs **410b,** and other data that may be needed to generate an RTTP (collectively patient data **410**) to the plan optimizer **430** to generate a suggested RTTP that is optimized for a patient and their treatment. The analytics server may also transmit the data **410** to the artificial intelligence computer model **420.** As a result, the artificial intelligence computer model **420** may transmit cost functions to the plan optimizer **430** where the two models can iteratively work together to generate the suggested treatment plan **440.**

The analytics server may first collect patient data **410.** The patient data may include patient anatomy data **410a** (e.g., medical images, PTVs, OARs), and user inputs **410b** (clinical objectives or rules received via a user interface from a treating oncologist, such as tumor data, PTV identification, and the like). Other non-limiting examples of clinical goals may include dosimetric goodness function, robustness metrics, biological effects of radiation, metrics of linear energy transfer, and the like. The patient data **410** may also include rules **410c** for the patient's treatment (e.g., clinical/treatment objectives or criteria identified by the medical professionals or any other special treatments required by the medical professionals).

In some configurations, the analytics server may access a patient's internal/external file and retrieve/extract the needed patient data **410.** The analytics server may then execute an artificial intelligence computer model **420** to identify a cost function for a candidate RTTP for the patient using the patient data **410.** The results generated via the artificial intelligence computer model **420** may be ingested by the plan optimizer **430.** The plan optimizer **430** may be a treatment planning and/or monitoring software solution. The plan optimizer **430** may analyze various factors associated with the patient and the patient's treatment to generate and optimize an RTTP for the patient (e.g., any combination of: field geometry, treatment modality, and radiation parameters needed to treat the patient).

One of the factors considered by the plan optimizer **430** may be the cost value calculated by the artificial intelligence computer model **420.** The plan optimizer **430** may iteratively revise the patient's RTTP wherein the plan optimizer **430** iteratively revises different attributes of the RTTP (e.g., field geometry). In some configurations, the plan optimizer **430** may transmit new treatment plan data back to the artificial intelligence model **420,** whereby the dose calculation sector model **420** can recalculate/re-predict the cost value based on the revised treatment data generated by the plan optimizer (iteration **422**). The plan optimizer **430** and the artificial intelligence model **420** may repeat iteration **422** until the patient's RTTP is optimized/finalized.

When the plan optimizer **430** completes the patient's RTTP, the plan optimizer **430** may transmit the suggested treatment plan **440** to one or more electronic devices where a user (e.g., clinician) can review the suggested plan. For instance, the suggested treatment plan **440** (or any attributes predicted by the dose calculation sector model **420**) may be displayed on a computer of a clinic where a radiation therapy technician or a treating oncologist can review the data.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or codes on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate the transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A method comprising:
receiving, by a processor, a radiation therapy plan objective for a patient;
executing, by the processor, a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value;
executing, by the processor, an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and
outputting, by the processor, the treatment plan for the patient.

2. The method of claim 1, further comprising:
transmitting, by the processor, the second cost value to the plan optimizer computer model, wherein the plan optimizer computer model revised the one or more attributes of the treatment plan in accordance with the second cost value.

3. The method of claim 1 or claim 2, wherein the health-problem corresponds to at least one of xerostomia, reduction of saliva production, headache, hair loss, nausea, vomiting, fatigue, hair loss, skin irritation, memory loss, or speech loss.

4. The method of any preceding claim, wherein the radiation therapy plan objective corresponds to a dose-volume objective;
and/or:
wherein the health-problem corresponds to developing a secondary cancer.

5. The method of any preceding claim, wherein the plan optimizer computer model aggregates the second cost value generated by the artificial intelligence model with the cost value generated by the plan optimizer computer model;
and/or:
wherein the artificial intelligence model is customized for a clinic implementing the treatment plan for the patient.

6. A non-transitory machine-readable storage medium having computer-executable instructions stored thereon that, when executed by one or more processors, cause the one or more processors to perform operations comprising:
receive a radiation therapy plan objective for a patient;
execute a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value;
execute an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and
output the treatment plan for the patient.

7. The non-transitory machine-readable storage medium of claim 6, wherein the instructions further cause the one or more processors to transmit the second cost value to the plan optimizer computer model, wherein the plan optimizer computer model revised the one or more attributes of the treatment plan in accordance with the second cost value.

8. The non-transitory machine-readable storage medium of claim 6 or claim 7, wherein the health-problem corresponds to at least one of xerostomia, reduction of saliva production, headache, hair loss, nausea, vomiting, fatigue, hair loss, skin irritation, memory loss, or speech loss.

9. The non-transitory machine-readable storage medium of any of claim 6 to claim 8, wherein the radiation therapy plan objective corresponds to a dose-volume objective;
and/or:
wherein the health-problem corresponds to developing a secondary cancer.

10. The non-transitory machine-readable storage medium of any of claim 6 to claim 9, wherein the plan optimizer computer model aggregates the second cost value generated by the artificial intelligence model with the cost value generated by the plan optimizer computer model;
and/or:
wherein the artificial intelligence model is customized for a clinic implementing the treatment plan for the patient.

11. A system comprising at least one processor configured to:
receive a radiation therapy plan objective for a patient;
execute a plan optimizer computer model to generate one or more treatment attributes for a treatment plan complying with the radiation therapy plan objectives, the plan optimizer computer model iteratively calculating the one or more attributes, where with each iteration, the plan optimizer computer model revises the one or more attributes of the treatment plan in accordance with a cost value;
execute an artificial intelligence model to calculate a second cost value for the treatment plan, wherein the artificial intelligence model is trained to calculate the second cost value in accordance with a likelihood of occurrence of a health-problem for the patient after being treated via the treatment plan having the one or more attributes; and
output the treatment plan for the patient.

12. The system of claim 11, wherein the at least one processor is further configured to transmit the second cost value to the plan optimizer computer model, wherein the plan optimizer computer model revised the one or more attributes of the treatment plan in accordance with the second cost value.

13. The system of claim 11 or claim 12, wherein the health-problem corresponds to at least one of xerostomia, reduction of saliva production, headache, hair loss, nausea, vomiting, fatigue, hair loss, skin irritation, memory loss, or speech loss.

14. The system of any of claim 11 to claim 13, wherein the radiation therapy plan objective corresponds to a dose-volume objective;
and/or:
wherein the health-problem corresponds to developing a secondary cancer.

15. The system of any of claim 11 to claim 14, wherein the plan optimizer computer model aggregates the second cost value generated by the artificial intelligence model with the cost value generated by the plan optimizer computer model.
